# EUROPEAN PATENT SPECIFICATION

(11) **EP 3 749 221 B1**
(45) Date of publication and mention of the grant of the patent: **03.01.2024**
(21) Application number: 19707704.3
(22) Date of filing: 06.02.2019
(51) Int. Cl.: A61B 17/02, A61B 17/00

(54) **SURGICAL TOOL**
CHIRURGISCHES INSTRUMENT
OUTIL CHIRURGICAL

(30) Priority: 06.02.2018 GB 201801892
(43) Date of publication of application: 16.12.2020
(73) Proprietor: Prosys International Limited, London SW19 1NE (GB)
(72) Inventor: NOE, Gunter, London SW19 1NE (GB)
(74) Representative: Bridge-Butler, Jeremy
(86) International application number: PCT/EP2019/052902
(87) International publication number: WO 2019/154852

(56) References cited:
- EP-A1- 2 578 167
- WO-A1-96/28099
- WO-A1-2012/087112
- WO-A1-2013/009795
- WO-A2-2013/010107

## Description

### Field

This invention relates to a surgical tool that is configured for insertion into a subject (for example via a trocar) during laparoscopic surgery (also otherwise known as "minimally invasive" or "keyhole" surgery).

In the following description of aspects of the invention, reference will be made to particular surgical procedures (for example, hysterectomies), but it will be appreciated by persons of ordinary skill in the art that the teachings of the invention are applicable to other surgical procedures, and thus the scope of the present invention should not be limited to the particular surgical procedures described herein.

### Background

In laparoscopic surgery it has previously been proposed to create a so-called pneumo-peritoneum in which the abdomen is expanded to thereby facilitate access to internal organs of the subject during the surgical procedure. In one arrangement, it has previously been proposed to pump carbon-dioxide into the abdominal cavity at a controlled pressure, typically at about 12-15 mm mercury.

It has also been proposed, for example for procedures in the lower part of the body, to shift the subject into a so-called "lithotomy" or "Trendelenburg" position in which the body is tilted in a head-down direction by 20 to 45 degrees. For procedures in the upper part of the body (for example, liver, gastric area or gall bladder procedures) it has previously been proposed to tilt the subject into a feet-down position known as a reverse Trendelenburg position.

These subject positions are adopted to facilitate the surgeon's access to the intended area of interest. In the context of pelvic surgery, the bowel (which moves continuously) is in danger of being injured by needles, scalpels, scissors or hot instruments during a given procedure. By adopting a Trendelenburg position for the subject, gravity assists the surgeon by tending to draw the bowel and other organs towards the head of the subject and thus out of the surgeon's way.

Obesity can reduce the effectiveness of the Trendelenburg position, as the nutritional area of the bowel tends to have more fatty tissue that hinders displacement of the bowel and other organs. It is also the case that the pressure of a higher fat load on the internal abdominal wall of obese subjects further hinders the effectiveness of the Trendelenburg position. The foregoing issues can complicate pelvic surgical procedures, and in some instances make such procedures entirely unfeasible.

To address such issues, it has previously been proposed to adopt an extreme Trendelenburg position (i.e. to increase the degree to which the subject is tilted), but such a position greatly increases the pressure in the head of the subject with potentially severe consequences. In addition, such a position can endanger subjects with vascular risks, as well as those with multiple co-morbidities (for example, blood pressure problems, reduced kidney function etc.).

If the Trendelenburg position is not sufficient to keep the bowel and other organs out of the surgeon's way or an extreme Trendelenburg position is not feasible due to the morbidity of the patient, the bowel has to be retracted by a grasper or retractor tools. Since the latter are only available as 10 mm instruments, larger trocars must necessarily be inserted into the subject. Graspers also increase the risk of inadvertently harming the bowel during the procedure as the tool will typically be out of sight.

As mentioned briefly above, extreme Trendelenburg positions can lead to many side effects. Redistribution of pooled venous blood from the lower limbs tends to increase venous return to the heart and thus increases cardiac output. This increased cardiac output can partially offset the cardiovascular depressant effects of many anaesthetic techniques. It is also the case that complications due to airway obstruction and decreased tidal volumes can occur.

For obese or pregnant subjects, severe hypotension may occur as a result of compression of the inferior vena cava against the vertebral bodies. In such circumstances, central redistribution of blood may lead to volume overload in the failing heart.

It is also the case that in supine patients, there is an increased risk of regurgitation of gastric contents, and diaphragmatic movement can be limited severely by the weight of the abdominal viscera which further reduces FRC (functional respiration capacity) and increases atelectasis. Ventilation-perfusion mismatch, raised intracranial pressure, raised intraocular pressure and passive regurgitation are all potential complications; the severity and likelihood of which increase with the amount of tilt.

As obesity is increasing in society, problems of the type aforementioned have become more commonplace. Aspects of the present invention have been devised with such problems in mind.

EP2578167A1 discloses various different hold back tools for laparoscopic insertion including a membrane that is surrounded by a frame. In some embodiments the frame is configured to be inflated with fluid in order to expand the frame.

### Summary

The present invention provides a surgical tool for laparoscopic insertion into a subject, the tool being expandable from a smaller collapsed configuration to a larger deployed configuration, the tool comprising an extendable barrier, an expandable support that is coupled to and surrounds the barrier, and a lumen having a distal end that is coupled to said support and in fluid communication with an internal void of said support; wherein said support can be expanded by injecting fluid into said void via said lumen and expansion of said support extends said barrier; characterised in that the barrier comprises two adjacent layers of flexible material, and further in that the barrier is provided with a plurality of weld lines that define a plurality of intersecting channels, each of said channels extending from one part of said support to a diametrically opposed part of said support and being in fluid communication with the internal void of said support.

The support preferably defines an aperture, the barrier being located in said aperture. The support is preferably annular, and the tool is generally circular or oval in said expanded configuration. Preferably the tool is suitable for insertion into a subject via a trocar when in said collapsed configuration. The support and barrier may be folded, furled or rolled together in said collapsed configuration.

The support may be inflatable via said lumen. The proximal end of said lumen remote from said support may include a coupling for connecting said lumen to a source of fluid for injection into said lumen. The coupling may include a valve.

The tool is preferably comprised of a biocompatible material. At least said barrier may be comprised of a material that is cut resistant and/or heat resistant.

Another aspect of the teachings of the invention comprises a surgical kit comprising: a surgical tool as described herein, and a trocar by means of which the tool can be laparoscopically inserted into a subject whilst the tool is in said collapsed configuration. The kit may include a syringe that is connectable to said support and is operable to expand said support. The kit may include an applicator for inserting the tool into a subject.

A further aspect of the invention provides a method of manufacturing a tool of the type described herein, comprising the steps of: forming a first component comprising a first barrier and a first half support extending around the first barrier; forming a second component comprising a second barrier and a second half support extending around the second barrier; and coupling the first and second components together so that the first and second barriers are adjacent and the first and second half supports form an expandable tubular body.

By adopting the teachings of the present invention, the tool can be used to keep the bowel and other organs out of the operative area and thereby facilitate access for the surgeon. The surgeon can work more effectively (saving time), and the risk of injury to the bowel can be reduced. The teachings of the invention also provide a tool that enables the surgeon to reduce the Trendelenburg position, thereby lowering general risks for subjects, and making surgery more available to high risk patients for whom such surgery was not previously feasible.

### Brief Description of the Drawings

Embodiments of the invention are illustrated in FIGS. 8-10. The arrangements as illustrated in FIGS. 5-7 do not form part of the invention. Various aspects of the disclosure will hereafter be described by way of illustrative example with reference to the accompanying drawings, in which:
Fig. 1 is a schematic representation of a surgical tool in a collapsed configuration;
Fig. 2 is a schematic representation of the tool depicted in Fig. 1 in a deployed configuration;
Fig. 3 is a diagrammatic sectional view of a supine subject with the tool of Fig. 2 inserted;
Fig. 4 is a partly cut-away diagrammatic plan view of the subject depicted diagrammatically in Fig. 3; and
Figs. 5 to 10 are schematic plan views of further tools that embody the teachings of the disclosure.

### Detailed Description

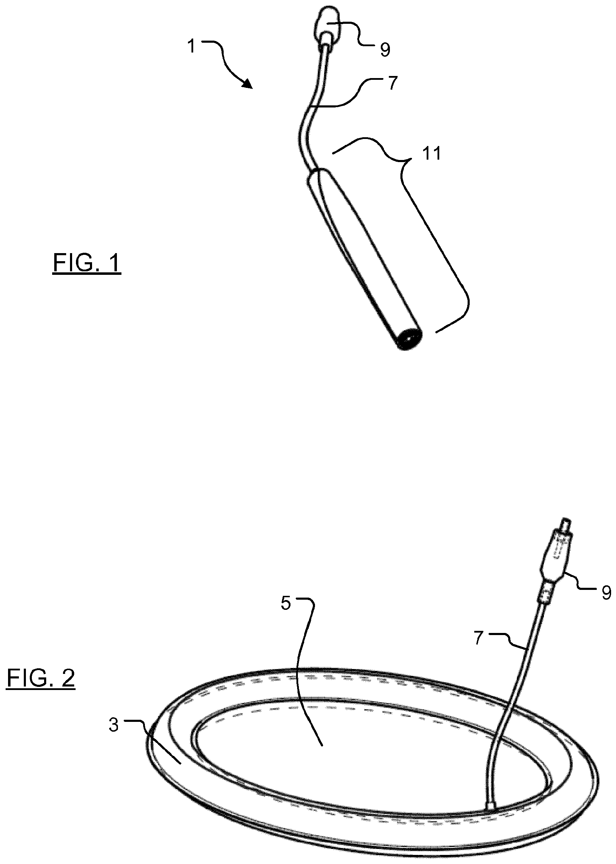

Figs. 1 and 2 of the accompanying drawings are diagrammatic representations of a surgical tool 1 in a collapsed and an expanded configuration, respectively.

Referring now to Figs. 1 and 2, the tool 1 comprises an expandable support 3 that is coupled to, and in this implementation, surrounds an extendable barrier 5. The expandable support comprises a tubular body that is in fluid communication with a lumen 7 which terminates at a fluid coupling 9. The fluid coupling 9 allows the lumen 7 (and hence the support 3) to be connected to a fluid pump (not shown) so that fluid can be pumped into the lumen to expand the support 3, and extend - and optionally tension - the barrier 5. The tool 1 is preferably circular when the support has been expanded and the barrier extended, but may conceivably be oval.

In one envisaged arrangement, the tool may be configured so that it can be unfurled by the surgeon whilst located inside the patient, and to this end may include one or more tabs (not shown) (for example coloured tabs that can more easily be located by the surgeon) that facilitate grasping of the tool by the surgeon using conventional laparoscopic tools. In another envisaged arrangement, the tool may be configured to automatically unfurl to extend the barrier as fluid is pumped into the extendable barrier. In yet another arrangement, the surgeon may partly unfurl the tool before expanding the support to extend the barrier. The tool may include one or more radio-opaque markers (not shown) to facilitate imaging of the tool within the subject.

In one envisaged implementation, the coupling may include a valve so that the fluid pump can be decoupled from the lumen once the support has been expanded. In another envisaged implementation, the fluid coupling may be without a valve and simply comprise a means for coupling the fluid pump to the support so that fluid may be pumped into and out of the support.

The support 3, barrier 5 and lumen are of a resilient, flexible biocompatible material, for example of silicone. At least the barrier 5 may be of a biocompatible material that is cut resistant and/or heat resistant. As shown in Fig. 1 the support 3 and barrier 5 can be rolled up into a cylindrical body 11 that is sized so that it can be inserted into the peritoneum of a subject, for example via a trocar. Alternatively, the surgeon may opt to manually poke the rolled up tool into the subject through an existing or new incision, for example an incision in the umbilicus. In one envisaged mode of operation, once the tool has been inserted and the fluid coupling 9 has been connected to a fluid pump, the pump can operated to pump fluid through the coupling 9 and into the lumen 7, whereupon the support 3 will start to expand and the cylindrical body will start to unfurl (and thereby starts to extend and deploy the barrier 5). Operation of the pump is continued until the tool 1 assumes the shape depicted in Fig. 2. In another envisaged mode of operation the tool may be partly or fully unfurled by the surgeon (using conventional laparoscopic tools) before the pump is operated to expand the support and extend the barrier.

In the preferred implementation, the support 3 is inflated by pumping a gas, for example Carbon Dioxide or Nitrogen, into the lumen 7, but it will be appreciated that other gases or fluids could instead be pumped into the support to extend and deploy the barrier 5.

In one envisaged mode of operation, at the start of a surgical procedure in which the tool 1 is to be used, the surgeon performing the procedure inserts a tool as depicted in Fig. 1 into the subject's inflated abdomen (for example via a trocar) so that the fluid coupling (and optionally some of the lumen) lies outside of the subject's body. The surgeon may then use conventional laparoscopic techniques to position the tool, and once properly positioned the surgeon can operate a fluid pump coupled to the fluid coupling to expand the support and thereby extend the barrier within the subject's peritoneum. The surgeon can then press the subject's internal organs aside (for example, by using a flat spatula), before placing the tool against the subject's organs so that those organs are kept clear from the area of the subject that the surgeon is going to operate on. To facilitate this part of the procedure, the surgeon may place the patient in the aforementioned Trendelenburg position, but in a departure from previous proposed techniques, once the surgical tool has been expanded and properly positioned within the subject, the extent to which the subject is inclined in the Trendelenburg position can be reduced and in some instances the subject can be returned to a conventional supine position.

In another mode of application, the surgeon may use conventional laparoscopic techniques to unfurl the tool within the patient, whereupon the support can then be expanded by means of the lumen that is at least partly outside of the subject's body. In yet another mode of application, the surgeon may insert the tool and then use conventional laparoscopic techniques to partly unfurl the tool, before expanding the support to extend the barrier.

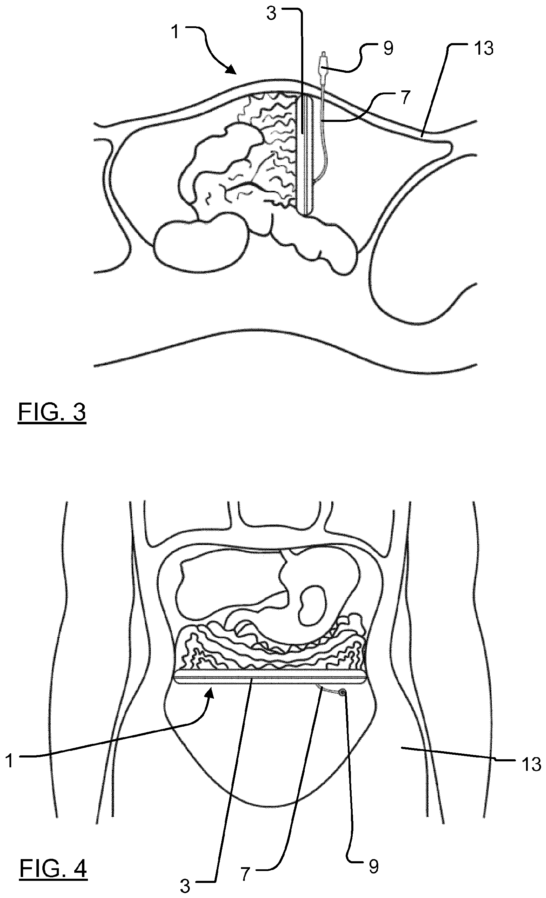

When positioned within the subject's body and in the expanded configuration, the internal pressure of the pneumo-peritoneum bears against the tool and helps keep the tool in a position where the subject's internal organs are displaced from the region in which the surgeon is to conduct the procedure. Figs. 3 and 4 are schematic cross-sectional side and part cut-away plan representations, respectively, of a subject 13 in whom a tool 1 has been inserted, unfurled, positioned and expanded/extended. When so arranged, the aforementioned Trendelenburg position can at least be reduced or in some instances altogether avoided.

More generally, it is the case that obstacles such as a moving bowel tend to be problematic during surgery. This is especially the case in laparoscopic surgery, in many different fields. Since many gynaecological procedures are performed by laparoscopy it is anticipated that the teachings of the present invention will have particular application in this field as such procedures previously tended to require that the patient is in a head down position in order to provide access to the region and the organs.

Illustrative procedures in which the tool may be employed are: Adnex removal, Tubal reconstruction, Adnex-cyst removal, ovary reconstruction, Hysterectomy (subtotal; total; and radical), Pelvic Lymphadenectomy, Fibroid resection, Uterus reconstruction, Colposuspension; Sacropexy; Pectopexy; laparoscopic lateral repair; laparoscopic anterior facia repair; laparoscopic posterior fascia repair; Laparoscopic recto-pexy; laparoscopic CESA/VASA; Peritoneal resection in the pelvis; Endometrioma removal; Bowel resection; ureter resection; de-bulking; adhesilyses; ureterolysis; bladder resection; ureterolysis; ureter implantation; Psoas-Hitch procedure; prostatectomy, urethra surgery; pelvic lymphnodes, Radical Hysterectomy; pelvic and para-aortal lymphadenectomy; radical resection of ovarian cancer; rectum cancer resection; sigmoid-resection, Rectum resection; and sigmoid resection.

In laparoscopic procedures, it is usually the case that the umbilicus is used to provide access for a camera as it is a natural scar and the smallest point of the abdominal wall. According to the preference of the surgeon 0-45 degree scopes with a camera are used to monitor the surgery via a display. Access ports of different diameter (5-10mm) are then placed by the surgeon in the lower abdomen. The placement of these ports depends on the predilections of the surgeon or special requirements of the procedure being undertaken.

One illustrative gynaecological procedure is the hysterectomy. This procedure starts with an incision in the umbilicus and dilatation of the fascia to provide a blind opening of the peritoneum. Next, a 10mm trocar is placed in the incision, and an insufflation tube (for example, CO₂) is connected. The peritoneum is then inflated under pressure control (12-15 mmHg) (approximately 1.8-3 litre CO₂), and a camera is introduced into the 10mm trocar so that the surgeon can visually inspect the complete abdominal area. The patient is then put into the Trendelenburg position (15-45 degrees) and access-ports are introduced in the lower abdomen. Usually two or three 5mm ports are inserted. The leading surgeon for example may then take a blunt grasper in his left hand and a coagulation grasper in his right hand. The assistant may have an additional grasper in his right hand and may control the camera with his left.

The procedure proper starts by heating the lateral upper ligaments (round ligament), after which the surgeon can cut the tissue with a scissor without bleeding. The peritoneum is then opened anteriorly in the direction of the bladder, and the connective tissue between the ovary and the uterus (ligament) is heated before being cut so that the ovary can be pushed away. The posterior peritoneum is then cut and opened completely, and the aforementioned steps are repeated for the other ovary.

The surgeon then locates the arteries that supply the region with blood and surrounding tissue is removed. The arteries are then heated and subsequently cut, before lower tissue is heated and dissected. The cup surrounding the cervix in the vagina is then pushed towards the surgeon so that the surgeon can identify the cup by touch.

The uterus is then pealed out of the lower connective tissue with a small hook. Monopolar energy heats the hook so that the surgeon can cut the tissue without causing bleeding. The dissection is performed straight to the rim of the cup to enable a close resection of the uterus without losing too much vaginal or connective tissue. The uterus is then removed vaginally
The vagina is then filled with a sponge or a small towel to avoid leakage of gas from the pneumo-peritoneum, and the vaginal cuff is closed by a suture (by single knots or a running suture). The area is then cleaned by irrigation and suction, following which the surgical field, bowel and both sides of the ureter are inspected.

Next the surgeon removes the access trocars, deflates the pneumo-peritoneum, and removes the camera port. The surgeon then sutures the fascia in the umbilicus, and the lower and umbilical incisions. Lastly, the surgeon disinfects the area (including the vagina) before applying sterile bandages.

In a typical hysterectomy procedure, the subject is in the Trendelenburg position for much of the procedure. By utilising a tool according to the teachings of the present invention, once the tool has been inserted and positioned the Trendelenburg position can be reduced, in some instances to zero.

Surgery in the upper part of the abdomen requires a reverse Trendelenburg position of the patient. Due to the presence of heavy fatty tissue obese subjects can be particularly difficult to operate on. A tool of the type described herein can improve access to the organs in this area. Endometriosis and some oncological procedures in gynaecology can also require surgery in the upper area. The tool described herein may also be useful in visceral surgery, for example in cholecystectomy; liver resection; gastric resection, diaphragm hernia, Omental resection and Spleen removal.

The tool described herein may be manufactured in a variety of different ways. In one envisaged arrangement, the support and barrier may be separately formed (for example by moulding), and then joined to one another (for example by welding). However, since separately moulding the two components may prove problematic, it is also envisaged to form the barrier and half of the support as a first component and the remaining half of the support as a second component that is then joined to the half support of the first component. In yet another arrangement it is proposed to form the tool as two components that each comprise a barrier arranged between two half supports, before subsequently joining the half support of one component to the half support of the other. An advantage of this last arrangement is that the tool would then comprise two adjacent barriers, and thus would be more resistant to inadvertent puncture by the surgeon than a tool with a single barrier.

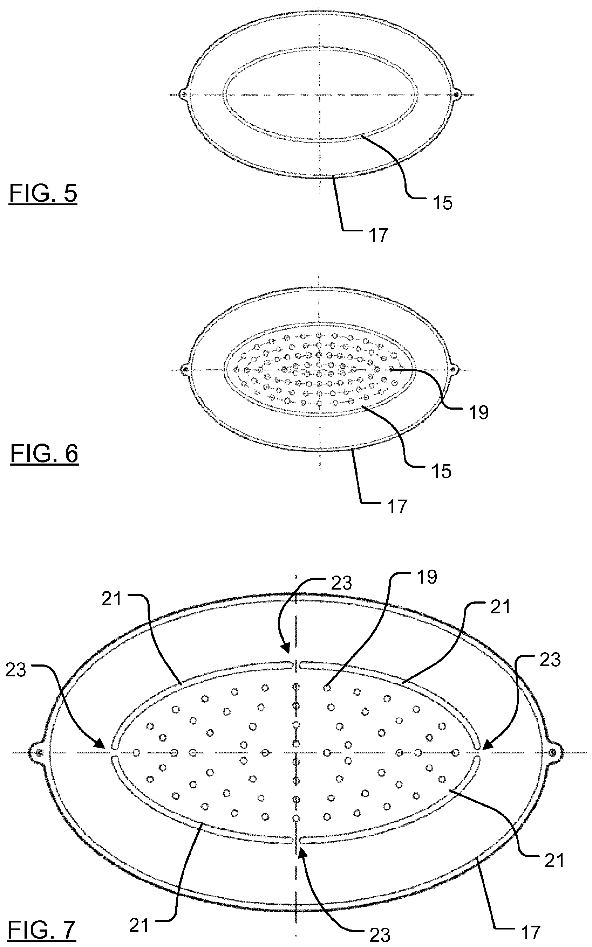

In the context of this last arrangement, a further advantage of having two components is that the components may be coupled together in a variety of different ways. For example, as depicted in Fig. 5, the two components could simply be coupled together by means of an inner weld line 15 and an outer weld line 17 to create a tubular body extending therebetween (which body can be inflated as previously described). In another envisaged arrangement depicted schematically in Fig. 6, the aforementioned inner and outer weld lines may be supplemented by a plurality of spot welds 19 which couple the two parts of the barrier together and help reduce the likelihood of the tool twisting as the support is inflated.

In a further arrangement, shown schematically in Fig. 7, four inner weld lines 21 are provided, with each said weld line being separated from adjacent weld lines so as to provide a channel 23 therebetween. As will be appreciated by persons skilled in the art, when the tool is inflated, fluid can pass through the aforementioned channels and into the space between the two parts of the barrier that are not coupled together by spot welds 19 to inflate the barrier and thereby reduce the likelihood of the tool twisting as it is inflated.

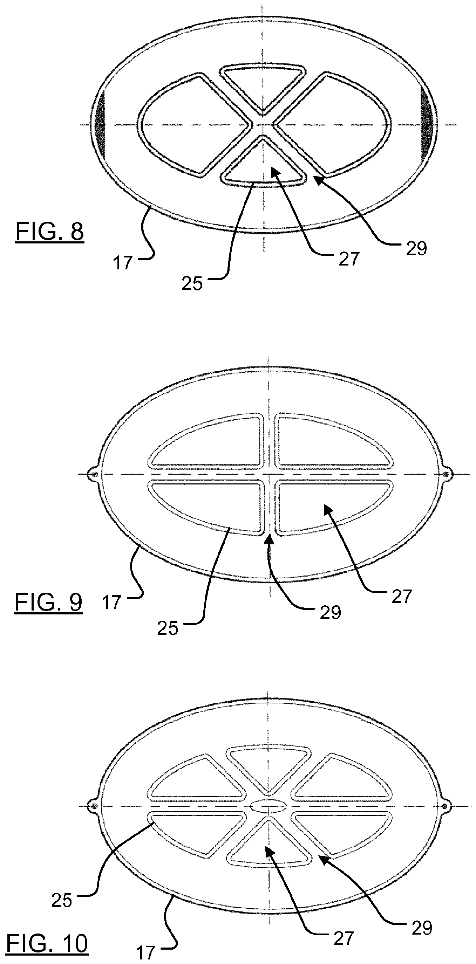

In arrangements that embody the teachings of the invention depicted schematically in Figs. 8 to 10, a plurality of inner weld lines 25 are provided, which weld lines divide the barrier into a plurality of regions 27 that are separated from one another by channels 29 which criss-cross through the barrier from one part of the support to a diametrically opposite part of the support, in a manner akin to the spokes of a bicycle wheel. In a similar manner to the arrangement shown in Fig. 7, when the tool is inflated, the channels 29 also inflate and thereby increase the tool's torsional rigidity to reduce the likelihood of the tool twisting as it is inflated. Figs. 8, 9 and 10 show, respectively, tools with two, two and three channels, although it will be appreciated that the number of channels may be varied as desired. In addition, it is also conceivable for the aforementioned regions 27 to include a plurality of spot welds, for example as shown in Figs. 6 and 7.

The tool may be packaged in its rolled up form (i.e. as shown in Fig.1), or in another envisaged arrangement the tool may be configured for use with an insertion device, for example a device akin to a conventional tampon applicator, and may be provided packaged with such a device ready for insertion into a subject.

It will be appreciated that whilst various aspects and embodiments of the present invention have heretofore been described, the scope of the present invention is not limited to the particular arrangements set out herein.

It should also be noted that whilst the accompanying claims set out particular combinations of features described herein, the scope of the present invention is not limited to the particular combinations hereafter claimed.

## Claims

1. A surgical tool (1) for laparoscopic insertion into a subject, the tool being expandable from a smaller collapsed configuration to a larger deployed configuration, the tool (1) comprising an extendable barrier (5), an expandable support (3) that is coupled to and surrounds the barrier (5), and a lumen (7) having a distal end that is coupled to said support (3) and in fluid communication with an internal void of said support (3); wherein said support (3) can be expanded by injecting fluid into said void via said lumen (7) and expansion of said support (3) extends said barrier (5); **characterised in that** the barrier (5) comprises two adjacent layers of flexible material, and further **in that** the barrier (5) is provided with a plurality of weld lines that define a plurality of intersecting channels (29), each of said channels (29) extending from one part of said support (3) to a diametrically opposed part of said support (3) and being in fluid communication with the internal void of said support (3).

2. A surgical tool (1) according to Claim 1, wherein the support (3) defines an aperture, the barrier (5) being located in said aperture.

3. A surgical tool (1) according to Claim 2, wherein the support (3) is annular, and the tool (1) is generally circular or oval in said expanded configuration.

4. A surgical tool (1) according to any preceding claim, wherein the tool (1) is suitable for insertion into a subject via a trocar when in said collapsed configuration.

5. A surgical tool (1) according to Claim 4, wherein said support (3) and barrier (5) are folded, furled or rolled together in said collapsed configuration.

6. A surgical tool (1) according to any preceding claim, wherein the support (3) is inflatable via said lumen (7).

7. A surgical tool (1) according to any preceding claim, wherein a proximal end of said lumen (7) remote from said support (3) includes a coupling (9) for connecting said lumen to a source of fluid for injection into said lumen.

8. A surgical tool (1) according to Claim 7, wherein said coupling (9) includes a valve.

9. A surgical tool (1) according to any preceding claim, wherein the tool (1) is comprised of a biocompatible material.

10. A surgical tool (1) according to any preceding claim, wherein at least said barrier (5) is comprised of a material that is cut resistant and/or heat resistant.

11. A surgical kit comprising: a surgical tool (1) according to any preceding claim, and a trocar by means of which the tool can be laparoscopically inserted into a subject whilst the tool is in said collapsed configuration.

12. A surgical kit according to Claim 11, further comprising a syringe that is connectable to said support and is operable to expand said support.

13. A surgical kit according to Claim 11 or 12, wherein said kit includes an applicator for inserting the tool (1) into a subject.

14. A method of manufacturing a tool (1) according to any of Claims 1 to 10, comprising the steps of: forming a first component comprising a first barrier and a first half support extending around the first barrier; forming a second component comprising a second barrier and a second half support extending around the second barrier; and coupling the first and second components together so that the first and second barriers are adjacent and the first and second half supports form an expandable tubular body.

## Patentansprüche

1. Chirurgisches Instrument (1) zur laparoskopischen Einführung in einen Patienten, wobei das Instrument von einer kleineren zusammengelegten Konfiguration zu einer größeren entfalteten Konfiguration aufpumpbar ist, wobei das Instrument (1) eine ausdehnbare Barriere (5), eine aufpumpbare Stütze (3), die mit der Barriere (5) verbunden ist und diese umgibt, und ein Lumen (7) umfasst, das ein distales Ende hat, das mit der Stütze (3) verbunden und in Fluidkommunikation mit einem inneren Hohlraum der Stütze (3) ist; wobei die Stütze (3) durch Einspritzen eines Fluids in den Hohlraum über das Lumen (7) aufgepumpt werden kann und das Aufpumpen der Stütze (3) die Barriere (5) ausdehnt; **dadurch gekennzeichnet, dass** die Barriere (5) zwei aneinander liegende Schichten eines flexiblen Werkstoffs umfasst, und ferner, dass die Barriere (5) mit einer Mehrzahl von Schweißnähten ausgestattet ist, die eine Mehrzahl sich kreuzender Kanäle (29) definieren, wobei jeder der Kanäle (29) sich von einem Teil der Stütze (3) zu einem diametral entgegengesetzten Teil der Stütze (3) erstreckt und mit dem inneren Hohlraum der Stütze (3) in Fluidkommunikation ist.

2. Chirurgisches Instrument (1) gemäß Anspruch 1, wobei die Stütze (3) eine Öffnung definiert, wobei die Barriere (5) in der Öffnung angeordnet ist.

3. Chirurgisches Instrument (1) gemäß Anspruch 2, wobei die Stütze (3) ringförmig ist und das Instrument (1) in der aufgepumpten Konfiguration allgemein kreisförmig oder oval ist.

4. Chirurgisches Instrument (1) gemäß einem der vorhergehenden Ansprüche, wobei das Instrument (1) zur Einführung in einen Patienten über einen Trokar geeignet ist, wenn es in der zusammengelegten Konfiguration ist.

5. Chirurgisches Instrument (1) gemäß Anspruch 4, wobei die Stütze (3) und die Barriere (5) in der zusammengelegten Konfiguration zusammengefaltet, zusammengerafft oder zusammengerollt sind.

6. Chirurgisches Instrument (1) gemäß einem der vorhergehenden Ansprüche, wobei die Stütze (3) über das Lumen (7) aufblasbar ist.

7. Chirurgisches Instrument (1) gemäß einem der vorhergehenden Ansprüche, wobei ein proximales Ende des Lumens (7), das von der Stütze (3) entfernt ist, eine Kupplung (9) aufweist, um das Lumen mit einer Quelle von Fluid zur Einspritzung in das Lumen zu verbinden.

8. Chirurgisches Instrument (1) gemäß Anspruch 7, wobei die Kupplung (9) ein Ventil aufweist.

9. Chirurgisches Instrument (1) gemäß einem der vorhergehenden Ansprüche, wobei das Instrument (1) aus einem biokompatiblen Material besteht.

10. Chirurgisches Instrument (1) gemäß einem der vorhergehenden Ansprüche, wobei mindestens die Barriere (5) aus einem Material besteht, das schnittfest und/oder wärmebeständig ist.

11. Chirurgischer Satz, umfassend: ein chirurgisches Instrument (1) gemäß einem der vorhergehenden Ansprüche, und einen Trokar, mittels dem das Instrument laparoskopisch in einen Patienten eingeführt werden kann, während das Instrument in der zusammengelegten Konfiguration ist.

12. Chirurgischer Satz gemäß Anspruch 11, ferner umfassend eine Kanüle, die mit der Stütze verbindbar und dazu betätigbar ist, die Stütze aufzupumpen.

13. Chirurgischer Satz gemäß Anspruch 11 oder 12, wobei der Satz einen Applikator zum Einführen des Instruments (1) in einen Patienten aufweist.

14. Verfahren zum Herstellen eines Instruments (1) gemäß einem der Ansprüche 1 bis 10, umfassend die folgenden Schritte:
Ausbilden einer ersten Komponente, die eine erste Barriere und eine erste Halbstütze umfasst, die sich um die erste Barriere herum erstreckt; Ausbilden einer zweiten Komponente, die eine zweite Barriere und eine zweite Halbstütze umfasst, die sich um die zweite Barriere herum erstreckt; und Verbinden der ersten und der zweiten Komponente miteinander, sodass die erste und die zweite Barriere aneinander liegen und die erste und die zweite Halbstütze einen aufpumpbaren rohrförmigen Körper bilden.

## Revendications

1. Un outil chirurgical (1) pour une insertion laparoscopique dans un sujet, l'outil étant expansible d'une configuration repliée plus petite à une configuration déployée plus grande, l'outil (1) comprenant une barrière extensible (5), un support extensible (3) qui est couplé à, et entoure, la barrière (5), et une lumière (7) ayant une extrémité distale qui est couplée audit support (3) et en communication fluidique avec un vide interne dudit support (3) ; dans lequel ledit support (3) peut être expandu en injectant un fluide dans ledit vide par l'intermédiaire de ladite lumière (7) et l'expansion dudit support (3) étend ladite barrière (5); **caractérisé en ce que** la barrière (5) comprend deux couches adjacentes de matériau flexible, et en outre **en ce que** la barrière (5) est pourvue d'une pluralité de lignes de soudure qui définissent une pluralité de canaux croisés (29), chacun desdits canaux (29) s'étendant d'une partie dudit support (3) à une partie diamétralement opposée dudit support (3) et étant en communication fluidique avec le vide interne dudit support (3).

2. Un outil chirurgical (1) selon la revendication 1, dans lequel le support (3) définit une ouverture, la barrière (5) étant située dans ladite ouverture.

3. Un outil chirurgical (1) selon la revendication 2, dans lequel le support (3) est annulaire, et l'outil (1) est généralement circulaire ou oval dans ladite configuration déployée.

4. Un outil chirurgical (1) selon l'une quelconque des revendications précédentes, dans lequel l'outil (1) est adapté pour être inséré dans un sujet par l'intermédiaire d'un trocart lorsqu'il est dans ladite configuration repliée.

5. Un outil chirurgical (1) selon la revendication 4, dans lequel ledit support (3) et ladite barrière (5) sont pliés, enroulés ou roulés ensemble dans ladite configuration repliée.

6. Un outil chirurgical (1) selon l'une quelconque des revendications précédentes, dans lequel le support (3) est gonflable par l'intermédiaire de ladite lumière (7).

7. Un outil chirurgical (1) selon l'une quelconque des revendications précédentes, dans lequel une extrémité proximale de ladite lumière (7) éloignée dudit support (3) comprend un raccord (9) pour connecter ladite lumière à une source de fluide pour l'injection dans ladite lumière.

8. Un outil chirurgical (1) selon la revendication 7, dans lequel ledit raccord (9) comprend une valve.

9. Un outil chirurgical (1) selon l'une quelconque des revendications précédentes, dans lequel l'outil (1) est composé d'un matériau biocompatible.

10. Un outil chirurgical (1) selon l'une quelconque des revendications précédentes, dans lequel au moins ladite barrière (5) est composée d'un matériau résistant à la coupure et/ou à la chaleur.

11. Un kit chirurgical comprenant : un outil chirurgical (1) selon l'une quelconque des revendications précédentes, et un trocart au moyen duquel l'outil peut être inséré par laparoscopie dans un sujet alors que l'outil est dans ladite configuration repliée.

12. Un kit chirurgical selon la revendication 11, comprenant en outre une seringue qui peut être connectée audit support et qui est actionnable pour étendre ledit support.

13. Un kit chirurgical selon la revendication 11 ou 12, dans lequel ledit kit comprend un applicateur pour insérer l'outil (1) dans un sujet.

14. Un procédé de fabrication d'un outil (1) selon l'une quelconque des revendications 1 à 10, comprenant les étapes de :
former un premier composant comprenant une première barrière et un premier demi-support s'étendant autour de la première barrière ; former un deuxième composant comprenant une deuxième barrière et un deuxième demi-support s'étendant autour de la deuxième barrière ; et coupler les premier et deuxième composants ensemble de telle sorte que les première et deuxième barrières sont adjacentes et que les premier et deuxième demi-supports forment un corps tubulaire extensible.
